# EUROPEAN PATENT SPECIFICATION

(11) **EP 3 843 829 B1**
(45) Date of publication and mention of the grant of the patent: **08.05.2024**
(21) Application number: 19804930.6
(22) Date of filing: 23.10.2019
(51) Int. Cl.: A61N 1/05, A61N 1/36

(54) **LEADS FOR STIMULATION AND SENSING IN A STIMULATOR DEVICE**
ELECTRODENLEITUNGEN ZUR STIMULATION UND MESSUNG IN EINER STIMULATIONSVORRICHTUNG
CONDUCTEURS DE STIMULATION ET DE DÉTECTION DANS UN DISPOSITIF STIMULATEUR

(30) Priority: 16.11.2018 US 201862768617 P
(43) Date of publication of application: 07.07.2021
(73) Proprietor: Boston Scientific Neuromodulation Corporation, Valencia, CA 91355 (US)
(72) Inventor: ESTELLER, Rosana, Santa Clarita, CA 91390 (US); CARBUNARU, Rafael, Valley Village, CA 91067 (US); FEATHERSTONE, Adam, Meridian, ID 83646 (US)
(74) Representative: Vossius & Partner Patentanwälte Rechtsanwälte mbB
(86) International application number: PCT/US2019/057631
(87) International publication number: WO 2020/101853

(56) References cited:
- US-A- 5 913 882
- US-A1- 2002 022 873
- US-A1- 2016 121 124
- US-A1- 2016 206 873
- US-A1- 2016 303 368

## Description

### FIELD OF THE INVENTION

The present invention relates generally to medical device systems, and more particularly to implantable stimulators with leads having electrodes to provide electrical stimulation and to sense a neural response to such stimulation.

### INTRODUCTION

Implantable stimulation devices deliver electrical stimuli to nerves and tissues for the therapy of various biological disorders, such as pacemakers to treat cardiac arrhythmia, defibrillators to treat cardiac fibrillation, cochlear stimulators to treat deafness, retinal stimulators to treat blindness, muscle stimulators to produce coordinated limb movement, spinal cord stimulators to treat chronic pain, cortical and Deep Brain Stimulators (DBS) to treat different neurological conditions including movement disorders, psychological disorders, migraine disorders, and epilepsy among others, and other neural stimulators to treat urinary incontinence, sleep apnea, shoulder subluxation, etc. The description that follows will generally focus on the use of the invention within a Spinal Cord Stimulation (SCS) system, such as that disclosed in U.S. Patent 6,516,227. However, the present invention may find applicability with any Implantable Pulse Generator (IPG) or in any IPG system.

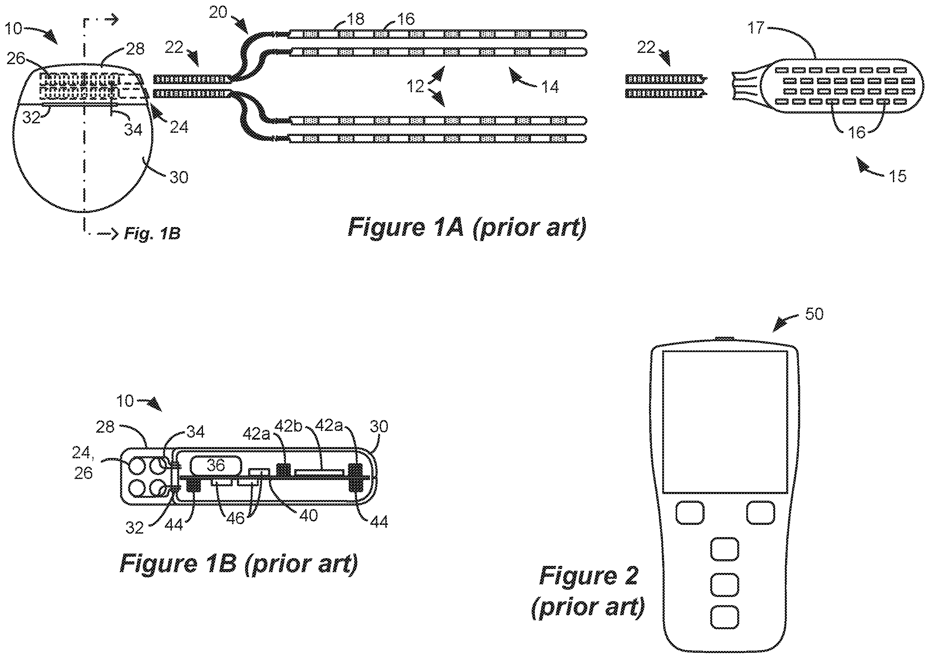

An SCS system typically includes an Implantable Pulse Generator (IPG) 10 shown in plan and cross-sectional views in Figures 1A and 1B. The IPG 10 includes a biocompatible device case 30 configured for implantation in a patient's tissue that holds the circuitry and battery 36 (Fig. 1B) necessary for the IPG to function. The IPG 10 is coupled to distal electrodes 16 via one or more electrode leads 14 that form an electrode array 12. The electrodes 16 are configured to contact a patient's tissue and are carried on a flexible body 18, which also houses the individual lead wires 20 coupled to each electrode 16. The lead wires 20 are also coupled to proximal contacts 22, which are insertable into lead connectors 24 fixed in a header 28 on the IPG 10, which header can comprise an epoxy for example. Once inserted, the proximal contacts 22 connect to header contacts 26 in the lead connectors 24, which are in turn coupled by electrode feedthrough pins 34 through an electrode feedthrough 32 to circuitry within the case 30 (connection not shown).

In the illustrated IPG 10, there are thirty-two lead electrodes (E1-E32) split between four leads 14, with the header 28 containing a 2x2 array of eight-electrode lead connectors 24 to receive the leads' proximal ends. However, the number of leads and electrodes in an IPG is application specific and therefore can vary. In a SCS application, the electrode leads 14 are typically implanted proximate to the dura in a patient's spinal cord, and when a four-lead IPG 10 is used, these leads can be split with two on each of the right and left sides. Two sixteen-electrode leads could also be used with each having two sets of proximal contacts 22 to allow the leads to be connected to two eight-electrode lead connectors 24. Each of the IPG's lead connectors 24 can also support differing numbers of electrodes, such as twelve or sixteen. The proximal contacts 22 of the leads 14 are tunneled through the patient's tissue to a distant location such as the buttocks where the IPG case 30 is implanted, at which point they are coupled to the lead connectors 24. As also shown in Figure 1A, one or more flat paddle leads 15 can also be used with IPG 10, and in the example shown thirty two electrodes 16 are positioned on one of the generally flat surfaces of the head 17 of the paddle lead, which surface would face the dura when implanted. In other IPG examples designed for implantation directly at a site requiring stimulation, the IPG can be lead-less, having electrodes 16 instead carried by the case of the IPG for contacting the patient's tissue. The conductive case 30 can also comprise an electrode.

As shown in the cross section of Figure 1B, the IPG 10 includes a printed circuit board (PCB) 40. Electrically coupled to the PCB 40 are the battery 36, which in this example is rechargeable; other circuitry 46 coupled to top and/or bottom surfaces of the PCB 40, including a microcontroller or other control circuitry necessary for IPG operation; and a charging coil 44 for wirelessly receiving a magnetic charging field from an external charger (not shown) for recharging the battery 36. If battery 36 is permanent and not rechargeable, charging coil 44 would be unnecessary.

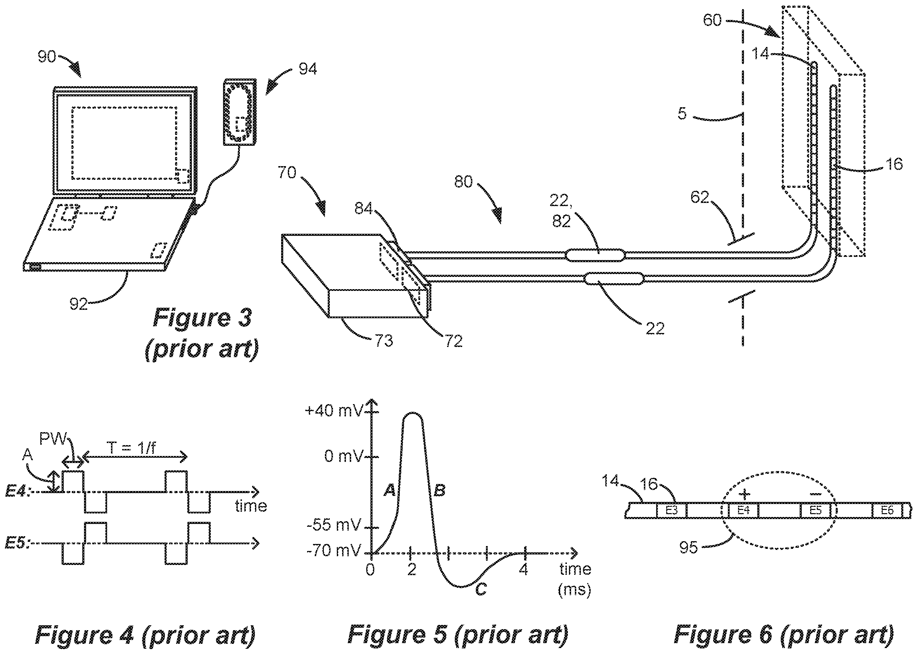

The IPG 10 also includes one or more antennas 42a and 42b for transcutaneously communicating with external programming devices, such as a patient external controller 50 (Fig. 2), or a clinician programmer 90 (Fig. 3). Antennas 42a and 42b are different in shape and in the electromagnetic fields they employ. Telemetry antenna 42a comprises a coil, which can bi-directionally communicate with an external device via a near-field magnetic induction communication link. Telemetry antenna 42b comprises a short-range RadioFrequency (RF) antenna that operates using far-field electromagnetic waves in accordance with a short-range RF communication standard, such as Bluetooth, BLE, NFC, Zigbee, and the Medical Implant Communication Service (MICS) or the Medical Device Radiocommunications Service (MDRS). Either of antennas 42a or 42b could appear within the case 30 or the header 28.

Implantation of IPG 10 in a patient is normally a multi-step process, as explained with reference to Figure 3. A first step involves implantation of the distal ends of the lead(s) 14 or 15 with the electrodes 16 into the spinal column 60 of the patient through a temporary incision 62 in the patient's tissue 5. (Only two leads 14 are shown in Figure 3 for simplicity). The proximal ends of the leads 14 or 15 including the proximal contacts 22 extend externally from the incision 62 (i.e., outside the patient), and are ultimately connected to an External Trial Stimulator (ETS) 70. The ETS 70 is used during a trial stimulation phase to provide stimulation to the patient, which may last for two or so weeks for example. To facilitate the connection between the leads 14 or 15 and the ETS 70, ETS extender cables 80 may be used that include receptacles 82 (similar to the lead connectors 24 in the IPG 10) for receiving the proximal contacts 22 of leads 14 or 15, and connectors 84 for meeting with ports 72 on the ETS 70, thus allowing the ETS 70 to communicate with each electrode 16 individually. Once connected to the leads 14 or 15, the ETS 70 can then be affixed to the patient in a convenient fashion for the duration of the trial stimulation phase, such as by placing the ETS 70 into a belt worn by the patient (not shown). ETS 70 includes a housing 73 for its control circuitry, antenna, etc., which housing 73 is not configured for implantation in a patient's tissue.

The ETS 70 essentially mimics operation of the IPG 10 to provide stimulation to the implanted electrodes 16, and thus includes contains a battery within its housing along with stimulation and communication circuitry similar to that provided in the IPG 10. Thus, the ETS 70 allows the effectiveness of stimulation therapy to be verified for the patient, such as whether therapy has alleviated the patient's symptoms (e.g., pain). Trial stimulation using the ETS 70 further allows for the determination of particular stimulation program(s) that seems promising for the patient to use once the IPG 10 is later implanted into the patient. A stimulation program may include stimulation parameters that specify for example: which of the electrodes 16 are to be active and used to issue stimulation pulses; the polarity of those active electrodes (whether they are to act as anodes or cathodes); the current or voltage amplitude (A) of the stimulation pulses; the pulse width (PW) of the stimulation pulses; the frequency (1) of the stimulation pulses; the duty cycle (DC) of the stimulation pulses (i.e., the percentage of time that the pulses are asserted relative to the period of the pulses); the shape of the stimulation waveform (e.g., one or more square pulses, one or more ramped pulses, one or more sinusoidal pulses, or even non-pulse-based waveforms, etc.); and other parameters related to issuing a burst of pulses, such as the number of pulses; etc.

At the end of the trial stimulation phase, a decision is made whether to abandon stimulation therapy, or whether to provide the patient with a permanent IPG 10 such as that shown in Figures 1A and 1B. Should it be determined that stimulation therapy is not working for the patient, the leads 14 or 15 can be explanted from the patient's spinal column 60 and incision 62 closed in a further surgical procedure. If stimulation therapy is effective, IPG 10 can be permanently implanted in the patient as discussed above. ("Permanent" in this context generally refers to the useful life of the IPG 10, which may be from a few years to a few decades, at which time the IPG 10 would need to be explanted and a new IPG 10 implanted). Thus, the IPG 10 would be implanted in the correct location (e.g., the buttocks) and connected to the leads 14 or 15, and then temporary incision 62 can be closed and the ETS 70 dispensed with. The result is fully-implanted stimulation therapy solution. If a particular stimulation program(s) had been determined as effective during the trial stimulation phase, it/they can then be programmed into the IPG 10, and thereafter modified wirelessly, using either the external programmer 50 or the clinician programmer 90.

An example of stimulation pulses as prescribed by a particular stimulation program and as executable by the IPG 10 or ETS 70 is illustrated in Figure 4. In the example shown, each stimulation pulse is biphasic, meaning it comprises a first pulse phase followed essentially immediately thereafter by an opposite polarity pulse phase. The pulse width (PW) could comprise the duration of either of the pulse phases individually as shown, or could comprise the entire duration of the biphasic pulse including both pulse phases. The frequency (f) and amplitude (A) of the pulses is also shown. Although not shown, monophasic pulses-having only a first pulse phase but not followed by an active-charge recovery second pulse phase-can also be used. In this circumstance, the first pulse phase may be followed by a passive charge recovery phase, as is known.

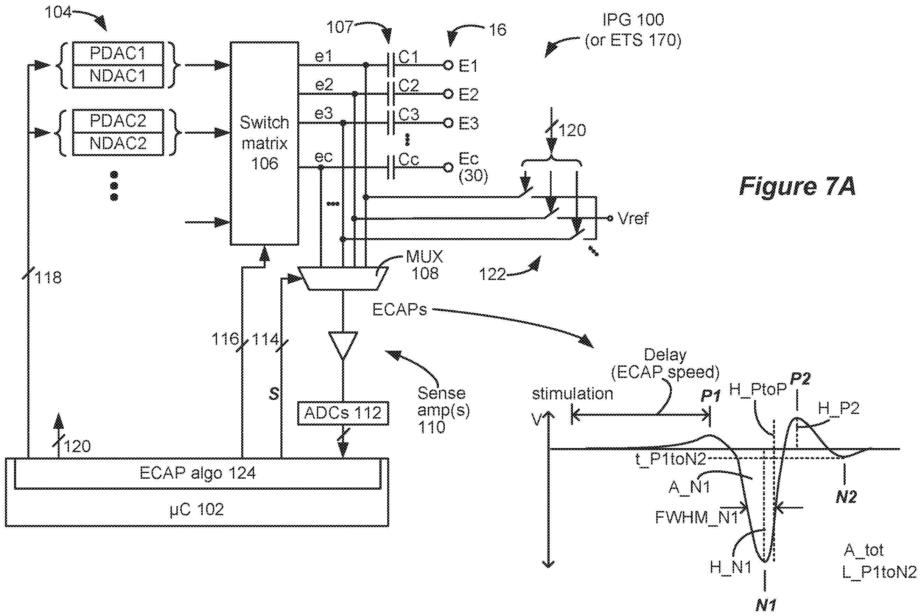

Biphasic pulses are useful because the second pulse phase can actively recover any charge build up after the first pulse phase residing on capacitances (such as the DC-blocking capacitors 107 discussed later with respect to Fig. 7A) in the current paths between the active electrodes. In the example stimulation program shown in Figure 4, electrode E4 is selected as the anode electrode while electrode E5 is selected as the cathode electrode (during the first pulse phase), which because two electrodes 16 are implicated, comprises what is known is bipolar stimulation. The pulses as shown comprise pulses of constant current, and notice that the amplitude of the current at any point in time is equal but opposite such that current injected into the patient's tissue by one electrode (e.g., E4) is removed from the tissue by the other electrode (E5). Notice also that the area of the first and second pulses phases are equal, ensuring active charge recovery of the same amount of charge during each pulse phase. Although not shown, more than two electrodes can be active at any given time. For example, electrode E4 could comprise a cathode providing a -I current pulse amplitude, while electrodes E3 and E5 could both comprise anodes with +0.51 current pulse amplitudes respectively, establishing a tripole.

The stimulation program executed by the IPG 10 and ETS 70 can be set or adjusted via a communication link from the external controller (Fig. 2) or a clinician programmer 90 (Fig. 3). While the external controller 50's antenna is usually within its housing, the clinician programmer 90 may include communication head or wand 94 containing an antenna and wired to computer 92. Further details concerning the clinician programmer 90 may be as described in U.S. Patent Application Publication 2015/0360038, and further details concerning an external controller can be found in U.S. Patent Application Publication 2015/0080982. As is known, both of the external communication devices have graphical user interfaces that can be used by the clinician or patient to set and adjust the stimulation program that the IPG 10 or ETS 70 will run.

US 2002/022873 A1 discloses leads with electrode arrays for neurostimulation.

### SUMMARY

A method for sensing a neural response caused by a stimulation is disclosed, which may comprise: providing from an implantable stimulator device the stimulation to one or more of a plurality first electrodes on a lead to cause the neural response in a patient's tissue; and sensing at the implantable stimulator device a neural response to the stimulation at two or more second electrodes on the lead; wherein the plurality of first electrodes are positioned along a long axis of the lead, wherein the first electrodes are spaced from one another by a first distance, wherein the at least two second electrodes are positioned such that one of the at least two second electrodes and one of the plurality of first electrodes are closest and spaced from each other by a second distance greater than the first distance, wherein the second distance is within a range of 15 mm to 40 mm, and wherein adjacent ones of the at least two electrodes are spaced from each other by a third distance, wherein the third distance is within a range of 4 mm to 28 mm. In one example, the second distance is within a range of 20 mm to less than 30 mm. In one example, the second distance is within a range of 8 mm to 17 mm. In one example, the plurality of first electrodes and the at least two second electrodes are located on a percutaneous portion of the lead. In one example, the at least two second electrodes comprise ring electrodes. In one example, the plurality of first electrodes comprise either ring electrodes or split ring electrodes. In one example, the at least two second electrodes are positioned along the long axis distally with respect to the plurality of first electrodes. In one example, the at least two second electrodes are positioned along the long axis proximally with respect to the plurality of first electrodes. In one example, the lead comprises a paddle, wherein the plurality of first electrodes are positioned on the paddle. In one example, the at least two second electrodes are positioned on a percutaneous lead portion positioned distally with respect to the paddle. In one example, the at least two second electrodes are positioned on a percutaneous lead portion positioned proximally with respect to the paddle. In one example, the at least two second electrodes are positioned on the paddle. In one example, the at least two second electrodes are positioned along the long axis.

A method for sensing a neural response caused by a stimulation is disclosed, which may comprise: providing from an implantable stimulator device the stimulation to one or more of a plurality first electrodes on a lead to cause the neural response in a patient's tissue; and sensing at the implantable stimulator device a neural response to the stimulation at either or both of at least one second electrode or at least one third electrode; wherein the plurality of first electrodes are positioned along a long axis of the lead, wherein the first electrodes are spaced from one another by a first distance, wherein the at least one second electrode is positioned distally with respect to the plurality of first electrodes such that one of the at least one second electrode and one of the plurality of first electrodes are closest and spaced from each other by a second distance greater than the first distance, and wherein the at least one third electrode is positioned proximally with respect to the plurality of first electrodes such that one of the at least one third electrode and one of the plurality of first electrodes are closest and spaced from each other by a third distance greater than the first distance. In one example, the plurality of first electrodes, the at least one second electrode, and the at least one third electrode are located on a percutaneous portion of the lead. In one example, the at least one second electrode and the at least one third electrode comprise a ring electrodes. In one example, the plurality of first electrodes comprise either ring electrodes or split ring electrodes. In one example, the second distance and the third distance are within a range of 15 mm to 40 mm. In one example, the second distance and the third distance are within a range of 20 mm to less than 30 mm. In one example, there is just one second electrode and just one third electrode. In one example, there are two or more second electrodes and/or two or more third electrodes. In one example, a distance between the two or more second electrodes and/or a distance between the two or more third electrodes is 4 to 28 mm. In one example, the distance between the two or more second electrodes and/or the distance between the two or more third electrodes is 8 to 17 mm. In one example, the lead comprises a paddle, wherein the plurality of first electrodes are positioned on the paddle. In one example, the at least one second electrode is positioned on a percutaneous lead portion positioned distally with respect to the paddle, and wherein the at least one third electrode is positioned on a percutaneous lead portion positioned proximally with respect to the paddle. In one example, the at least one second electrode and the at least one third electrode are positioned on the paddle. In one example, the at least one second electrode and the at least one third electrode are positioned along the long axis.

A system is disclosed, which may comprise: a lead for an implantable stimulator device, comprising: a plurality of first electrodes positioned along a long axis of the lead, wherein the first electrodes are spaced from one another by a first distance, wherein the first electrodes are configured to provide stimulation to a patient's tissue to thereby create a neural response; and at least two second electrodes positioned such that one of the at least two second electrodes and one of the plurality of first electrodes are closest and spaced from each other by a second distance greater than the first distance, wherein the at least two second electrodes are configured to sense the neural response, wherein the second distance is within a range of 15 mm to 40 mm, and wherein adjacent ones of the at least two electrodes are spaced from each other by a third distance, wherein the third distance is within a range of 4 mm to 28 mm. In one example, the second distance is within a range of 20 mm to less than 30 mm. In one example, the second distance is within a range of 8 mm to 17 mm. In one example, the plurality of first electrodes and the at least two second electrodes are located on a percutaneous portion of the lead. In one example, the at least two second electrodes comprise ring electrodes. In one example, the plurality of first electrodes comprise either ring electrodes or split ring electrodes. In one example, the at least two second electrodes are positioned along the long axis distally with respect to the plurality of first electrodes. In one example, the at least two second electrodes are positioned along the long axis proximally with respect to the plurality of first electrodes. In one example, the lead comprises a paddle, wherein the plurality of first electrodes are positioned on the paddle. In one example, the at least two second electrodes are positioned on a percutaneous lead portion positioned distally with respect to the paddle. In one example, the at least two second electrodes are positioned on a percutaneous lead portion positioned proximally with respect to the paddle. In one example, the at least two second electrodes are positioned on the paddle. In one example, the system further comprises the implantable stimulator device, wherein the lead further comprises at least one set of proximal contacts configured to be received by the implantable stimulator device, wherein the proximal contacts are connected to the plurality of first electrodes and to the at least two second electrodes by wires. In one example, the implantable stimulator device comprises an architecture, wherein the architecture is configured to allow the plurality of first electrodes to be selected to either provide stimulation to a patient's tissue or to sense the neural response, and configured to allow the at least two second electrodes to be selected to either provide stimulation to a patient's tissue or to sense the neural response. In one example, the implantable stimulator device comprises an architecture, wherein the architecture is configured to allow the plurality of first electrodes to be selected to only provide stimulation to a patient's tissue, and configured to allow the at least two second electrodes to be selected to only sense the neural response. In one example, the at least two second electrodes are positioned along the long axis.

A system is disclosed, which may comprise: a lead for an implantable stimulator device, comprising: a plurality of first electrodes positioned along a long axis of the lead, wherein the first electrodes are spaced from one another by a first distance, wherein the first electrodes are configured to provide stimulation to a patient's tissue to thereby create a neural response; at least one second electrode positioned distally with respect to the plurality of first electrodes such that one of the at least one second electrode and one of the plurality of first electrodes are closest and spaced from each other by a second distance greater than the first distance, wherein the at least one second electrode is configured to sense the neural response; and at least one third electrode positioned proximally with respect to the plurality of first electrodes such that one of the at least one third electrode and one of the plurality of first electrodes are closest and spaced from each other by a third distance greater than the first distance, wherein the at least one third electrode is configured to sense the neural response. In one example, the plurality of first electrodes, the at least one second electrode, and the at least one third electrode are located on a percutaneous portion of the lead. In one example, the at least one second electrode and the at least one third electrode comprise a ring electrodes. In one example, the plurality of first electrodes comprise either ring electrodes or split ring electrodes. In one example, the second distance and the third distance are within a range of 15 mm to 40 mm. In one example, the second distance and the third distance are within a range of 20 mm to less than 30 mm. In one example, there is just one second electrode and just one third electrode. In one example, there are two or more second electrodes and/or two or more third electrodes. In one example, a distance between the two or more second electrodes and/or a distance between the two or more third electrodes is 4 to 28 mm. In one example, the distance between the two or more second electrodes and/or the distance between the two or more third electrodes is 8 to 17 mm. In one example, the lead comprises a paddle, wherein the plurality of first electrodes are positioned on the paddle. In one example, the at least one second electrode is positioned on a percutaneous lead portion positioned distally with respect to the paddle, and wherein the at least one third electrode is positioned on a percutaneous lead portion positioned proximally with respect to the paddle. In one example, the at least one second electrode and the at least one third electrode are positioned on the paddle. In one example, the system further comprises the implantable stimulator device, wherein the lead further comprises at least one set of proximal contacts configured to be received by the implantable stimulator device, wherein the proximal contacts are connected to the plurality of first electrodes, to the at least one second electrode, and to the at least one third electrode by wires. In one example, the implantable stimulator device comprises an architecture, wherein the architecture is configured to allow the plurality of first electrodes to be selected to either provide stimulation to a patient's tissue or to sense the neural response, and configured to allow the at least one second electrode and the least one third electrode to be selected to either provide stimulation to a patient's tissue or to sense the neural response. In one example, the implantable stimulator device comprises an architecture, wherein the architecture is configured to allow the plurality of first electrodes to be selected to only provide stimulation to a patient's tissue, and configured to allow the at least one second electrode and the at least one third electrode to be selected to only sense the neural response. In one example, the at least one second electrode and the at least one third electrode are positioned along the long axis.

### BRIEF DESCRIPTION OF THE DRAWINGS

Figures 1A and 1B respectively show an Implantable Pulse Generator (IPG) in plan and cross sectional views, in accordance with the prior art.
Figure 2 shows a hand-held external controller for communicating with an IPG, in accordance with the prior art.
Figure 3 shows a clinician programming system for communicating with an IPG or an External Trial Stimulator (ETS), in accordance with the prior art.
Figure 4 shows pulses in a stimulation program, in accordance with the prior art.
Figure 5 shows a graph of an action potential of a neuron, in accordance with the prior art.
Figure 6 shows an electromagnetic field produced in a patient's tissue for recruiting neurons to fire, in accordance with the prior art.
Figure 7A shows an improved architecture for an IPG (or ETS) including control circuitry programmed with an Evoked Compound Action Potential (ECAP) algorithm, and further including sensing circuitry for sensing an ECAP at a sense electrode.
Figure 7B shows sense amplifier circuitry useable in the improved architecture for sensing an ECAP at one or more sense electrodes.
Figures 8A and 8B show a stimulation program, a stimulation artifact resulting from that program, the resulting generation of an ECAP, and detection of that ECAP by the ECAP algorithm in the improved IPG (or ETS).
Figures 9A and 9B show how amplitudes of ECAPs and stimulation artifacts vary as a function of a distance between a stimulus and the location at which the ECAP is sensed, while Figure 9C shows how the amplitudes of ECAPs vary as a function of the distance between sensing electrodes.
Figure 10 shows various examples of eight-electrode percutaneous leads in accordance with the invention, in which one or more sensing electrodes are provided along with one or more sensing electrodes, and in which the sensing electrodes are spaced from the sensing electrodes by a preferred distance at which ECAPs can be sensed without significant interference from the stimulation artifact.
Figure 11 shows various examples of sixteen-electrode percutaneous leads in accordance with the invention, in which one or more sensing electrodes are provided along with one or more sensing electrodes, and in which the sensing electrodes are again spaced from the sensing electrodes by a preferred distance.
Figure 12 shows various examples of sixteen-electrode leads incorporating a paddle in accordance with the invention, in which one or more sensing electrodes are provided along with one or more sensing electrodes, and in which the sensing electrodes are spaced from the sensing electrodes by a preferred distance at which ECAPs can be sensed without significant interference from the stimulation artifact.
Figure 13 shows another example of an improved architecture for an IPG (or ETS) including control circuitry programmed with an Evoked Compound Action Potential (ECAP) algorithm, in which the sensing electrodes are dedicated to providing sensing and in which the stimulating electrodes are dedicated to providing stimulation.

### DETAILED DESCRIPTION

When a neural fiber is recruited by electrical stimulation, it will issue an action potential-that is, the neural fiber will "fire." An action potential for a typical neural fiber is shown in Figure 5. Should electrical recruitment from electrical stimulation cause the neural fiber's resting state (e.g., -70mV as measured from inside the cell) to exceed a threshold (e.g., -55mV), the neural fiber will depolarize ("A"), repolarize ("B"), and hyperpolarize ("C") before coming to rest again. If electrical stimulation continues, the neural fiber will fire again at some later time. Note that the action potential does not change in magnitude for a given neural fiber. Instead, changing the strength of stimulation may affect the frequency at which action potentials are issued, and may also affect what types of neural fibers are recruited. Each neural fiber is unique in its shape and size, and thus can fire at its own inherent maximum frequency.

Figure 6 illustrates the stimulation program example of Figure 4 in which electrodes E4 and E5 on lead 14 are used to produce pulses in a bipolar mode of stimulation, with E4 comprising an anode (+; or source of current) and E5 a cathode (-; or sink of current). Such stimulation produces an electromagnetic (EM) field in a volume 95 of the patient's tissue around the selected electrodes. Some of the neural fibers within the EM field volume 95 will be recruited and fire, particularly those proximate to the cathodic electrode E5. Hopefully the sum of the neural fibers firing within volume 95 will mask signals indicative of pain in an SCS application, thus providing the desired therapy.

Neural fibers recruited and that fire within volume 95 in response to the stimulation create a cumulative response called an Evoked Compound Action Potential, or ECAP, which is shown in Figure 7A along with circuitry for an IPG 100 or ETS 170 capable of sensing the ECAP. The IPG 100 (or ETS 170) includes control circuitry 102 into which an ECAP algorithm 124 can be programmed. Control circuitry 102 may comprise a microcontroller for example such as Part Number MSP430, manufactured by Texas Instruments, which is described in data sheets at http://www.ti.com/ lsds/ ti/ microcontroller/ 16-bit_msp430/ overview.page? DCMP = MCU_other& HQS = msp430. Other types of control circuitry may be used in lieu of a microcontroller as well, such as microprocessors, FPGAs, DSPs, or combinations of these, etc. Control circuitry 102 may also be formed in whole or in part in one or more Application Specific Integrated Circuits (ASICs), as described in U.S. Patent Application Publication 2012/0095529 and USPs 9,061,140 and 8,768,453. One skilled in the art will understand that the ECAP algorithm 124 and/or any supporting user interface program will comprise instructions that can be stored on non-transitory machine-readable media, such as magnetic, optical, or solid-state memories. Such memories may be within the IPG or ETS itself (i.e., stored in association with control circuitry 102), within the external system (e.g., 50 or 90), or readable by the external system (e.g., memory sticks or disks). Such memories may also include those within Internet or other network servers, such as an implantable medical device manufacturer's server or an app store server, which may be downloaded to the external system.

In the IPG 100 or ETS 170, a bus 118 provides digital control signals to one or more Digital-to-Analog converters (DACs) 104, which are used to produce currents or voltages of prescribed amplitudes (A) for the stimulation pulses, and with the correct timing (PW, f). As shown, the DACs include both PDACs which source current to one or more selected anode electrodes, and NDACs which sink current from one or more selected cathode electrodes. In this example, a switch matrix 106 under control of bus 116 is used to route the output of one or more PDACs and one or more NDACs to any of the electrodes, including the conductive case electrode 30 (Ec), which effectively selects the active anode and cathode electrodes. Buses 118 and 116 thus generally set the stimulation program the IPG 100 is running. The illustrated circuitry for producing stimulation pulses and delivering them to the electrodes is merely one example. Other approaches may be found for example in USPs 8,606,362 and 8,620,436, and U.S. Patent Application Publication 2018/0071520. Note that a switch matrix 106 isn't required, and instead a PDAC and NDAC can be dedicated to (e.g., wired to) each electrode, as shown for example in USP 6,181,969. The PDACs and NDACs may more generally be referred to as current sources, and may output a constant current or voltage.

Notice that the current paths to the electrodes 16 include the DC-blocking capacitors 107 alluded to earlier, which provide additional safety by preventing the inadvertent supply of DC current to an electrode and to a patient's tissue. As discussed earlier, capacitances such as these can become charged as stimulation currents are provided, providing an impetus for the use of biphasic pulses.

One or more of the electrodes 16, again including the case electrode Ec, can be used to sense the ECAP described earlier, and thus each electrode in this example is further coupleable to at least one sense amp 110. In one example, there are four sense amps 110 each corresponding to a particular timing channel in which stimulation can be issued. Under control by bus 114, a multiplexer 108 can couple any of the electrodes to any of the sense amps 110 at a given time. This is however not strictly necessary, and instead each electrode can be coupleable to its own dedicated sense amp 110, or all electrodes can be selected for sensing at different times and presented by MUX 108 to a single sense amp 110. The analog waveform comprising the ECAP, described further below, is preferably converted to digital signals by one or more Analog-to-Digital converters (ADC(s)) 112, which may sample the waveform at 50 kHz for example. The ADC(s) may also reside within the control circuitry 102, particularly if the control circuitry 102 has A/D inputs.

Notice that connection of the electrodes 16 to the sense amp(s) 110 preferably occurs through the DC-blocking capacitors 107, such that capacitors 107 are between the electrodes (Ei) and corresponding electrode nodes (ei) that are presented to the sense amp(s) 110. This is preferred so as to not undermine the safety provided by the DC-blocking capacitors 107. The DC-blocking capacitor 107 will remove any DC components in the detected ECAP signals, which are thus referenced to 0 Volts. If necessary, the sensed ECAP signal can be amplified and level-shifted by the sense amp(s) 110 so that its voltage is brought within a range that the control circuitry 102 and/or ADCs 112 can handle, such as between 3 Volts and ground. Alternatively, sensing of neural responses may occur without the signals passing through the DC-blocking capacitors 107, and thus the voltages at the electrode Ei can be presented direct to the sense amp(s) 110.

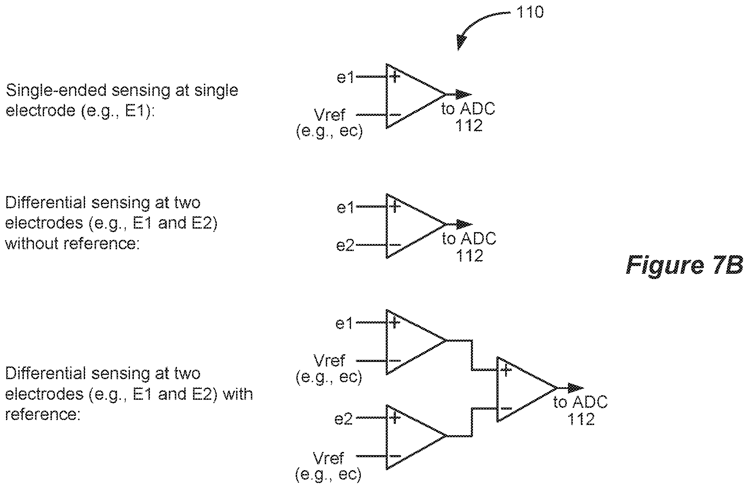

Figure 7B shows further details of different examples of the sense amp circuitry 110. and illustrates that sensing of a neural response can occur using a single electrode (single-ended sensing) or more than one electrode (differential sensing). In a first example at the top of Figure 7B, single-ended sensing is used to sense the ECAP at a selected sensing electrode, assumed in this example to be electrode E1. The corresponding electrode node voltage e1 (on the other side of DC-blocking capacitor 107 if used) is presented to a differential amplifier, and compared to a reference voltage, Vref. Vref can comprise any suitable reference voltage, such as ground or another constant voltage providable by the circuitry. The reference voltage can comprise the voltage at the case electrode, e.g., Ec (30), or as shown its corresponding voltage ec on the other side of a DC-blocking capacitor 107 if used. This reference voltage may not be constant, but nonetheless can be particularly useful, because any background signals in the tissue, such as the stimulation artifact 131 described later, may be present at both Ec and E1, and thus subtracted out by the differential amplifier, leaving at the output only the ECAP.

The next example shows differential sensing at two selected sensing electrodes E1 and E2. Thus, corresponding voltages e1 and e2 are sent to the differential amplifier, which will output the difference sensed between the electrodes. Because the background signal in the tissue would be present at both selected electrodes, they would again be subtracted from the sensed difference, which would therefore include just the ECAP neural response. Note, and as discussed further below, that neural response will move through the neural environment, and will thus be presented to the two different sensing electrodes at different times. If the distance between the sensing electrodes is known, this timing difference allows the speed of the neural response to be calculated. Note that sensing electrodes could also appear on different leads, although this isn't illustrated.

The last example also involves differential sensing between two selected sensing electrodes E1 and E2, but also uses a reference voltage Vref, similarly to what was used in single-ended sensing at a single electrode. Again, Vref can comprise a constant voltage, or a varying voltage such as the voltage at the case electrode Ec (or ec). Two first differential amplifiers are used to essentially single-ended sense the neural response at each electrode. The outputs of these two first differential amplifiers are then provided to a second differential amplifier to provide the difference. This arrangement may be more effective in reducing the background signal, as it can be subtracted from both the two first differential amplifiers and the second differential amplifier.

Once the digitized ECAP is received at the control circuitry 102, it is processed by the ECAP algorithm 124 to determine one or more ECAP features that describe the basic shape and size of the ECAP(s). As shown, peaks in the ECAP are conventionally labeled with P for positive peaks and N for negative peaks, with P1 comprising a first positive peak, N1 a first negative peak, P2 a second positive peak and so on. Note that not all ECAPs will have the exact shape and number of peaks as illustrated in Figure 7A, because an ECAP's shape is a function of the number and types of neural fibers that are recruited in a given volume 95. Various features for an ECAP are shown in Figure 7A, which include but are not limited to:
- a height of any peak (e.g., H_N1) present in the ECAP;
- a peak-to-peak height between any two peaks (such as H_PtoP from N1 to P2);
- a ratio of peak heights (e.g., H_N1 / H_P2);
- a peak width of any peak (e.g., the full width half maximum of a N1, FWHM_N1);
- an area under any peak (e.g., A_N1);
- a total area (A_tot) comprising the area under positive peaks with the area under negative peaks subtracted or added;
- a length of any portion of the curve of the ECAP (e.g., the length of the curve from P1 to N2, L_P1toN2)
- any time defining the duration of at least a portion of the ECAP (e.g., the time from P1 to N2, t_P1toN2);
- a time delay from stimulation to issuance of the ECAP, which is indicative of the neural conduction speed of the ECAP, which can be different in different types of neural tissues;
- any mathematical combination or function of these variables (e.g., H_N1 / FWHM_N1 would generally specify a quality factor of peak N1).

Once the ECAP algorithm 124 assesses one or more of these features, it may then adjust the stimulation that the IPG 100 or ETS provides. This is explained further in U.S. Patent Application Publications 2017/0296823 and 2019/0099602.

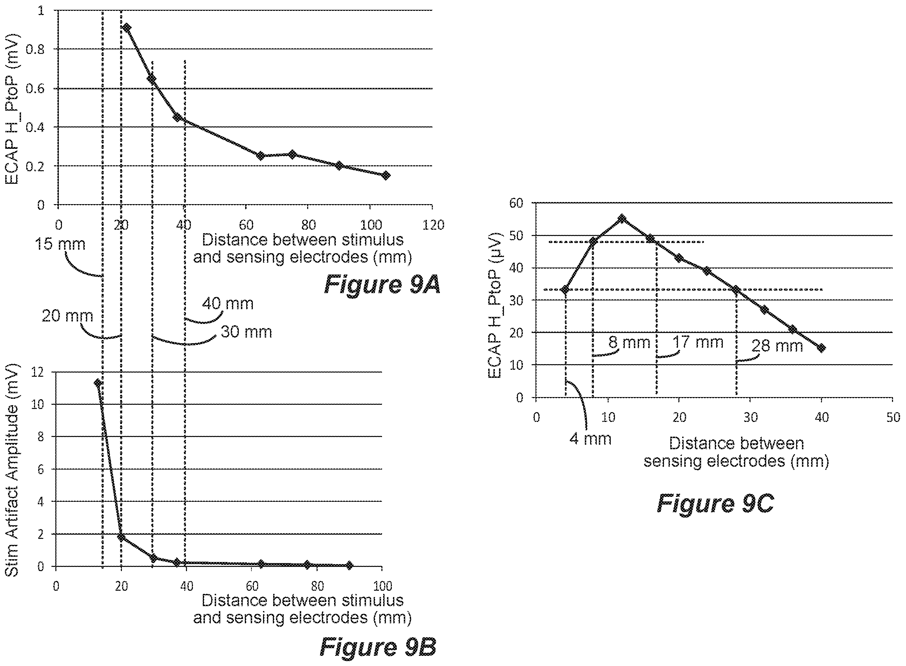

The amplitude of an ECAP will depend on how many neural fibers are firing. Generally speaking, a primary ECAP response, such as the height between peaks N1 and P2 (H_PtoP) can vary, is usually between microVolts to tens of millivolts. The amplitude of an ECAP will also depend on how far away the ECAP is sensed from the stimulus that created it. This is shown in Figure 9A, which shows experimental data taken in porcine spinal cord tissue. Generally speaking, the amplitude of an ECAP is largest near the source of the stimulus, but declines as the ECAP moves away from this stimulus, because the signal spreads out in the tissue at greater distances.

An ECAP can comprise the summation of action potentials observed at different delays corresponding to different types of neural elements recruited. Neural elements include axon fibers, neuron cell bodies, neuron dendrites, axon terminals, locations where fiber collaterals branch, interneurons, glial cells, or any nervous system functional part. In the specific case of the spinal cord, the sense electrodes can be placed over the dorsal column, more laterally in the epidural space towards and over the edge of dorsal horn and/or Lissauer's tract, over the dorsal root entry zone (DREZ), the rootlets, the dorsal root ganglia (DRG), the cauda equina region, the spinal nerves close to the spinal cord, the Spino-thalamic tract, and any other of the tracts surrounding the gray matter of the spinal cord. An ECAP can contain a number of peaks as noted earlier, and peak potentials can be indicative of different type of fibers activated. Also, axon fibers with different functions (C fibers, Aβ fibers, Aδ fibers, and others) have different diameters that correlate with different propagation speeds for the ECAPs. For example, ECAPs can travel at speeds of about 3.5-7.5 cm/ms in the typical case of Aβ fibers, or 0.3-3.0 cm/ms in the case of Aδ fibers. The conduction speed of the ECAPs sensed in the spinal cord can be calculated by the ECAP algorithm 124 to determine the originating fiber.

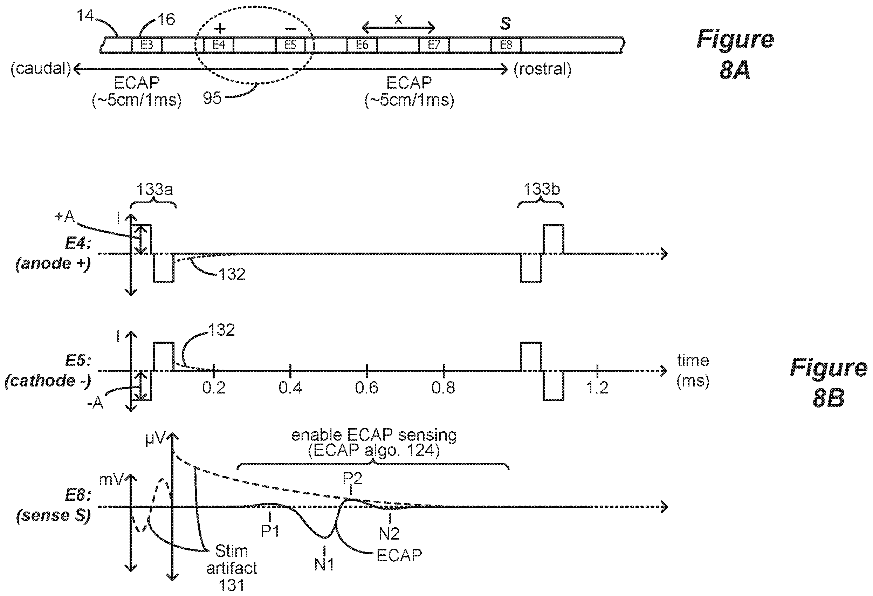

Figures 8A and 8B illustrate a particular stimulation program, the resulting generation of an ECAP, and detection of that ECAP. The stimulation program is defined as before by various stimulation parameters that form stimulation pulses 133i, such as which electrodes are active for stimulation, the polarity of those electrodes, the amplitude at selected electrodes, pulse width, pulse frequency, and stimulation waveform shape (square pulses in the example shown), although these parameters are not all labeled in Figure 8B. In the example stimulation program shown, biphasic pulses 133i are used, each having a duration of about 0.1 ms. Considering only the first phase of the biphasic pulses, electrode E4 is selected to operate as an anode (+), and electrode E5 is selected to operate a cathode (-). It is assumed that this particular stimulation program has been chosen as one that generally provides good therapeutic results for a particular patient. Note that the pulse phases in Figure 8B could also comprise a burst of (higher-frequency) pulse phases, although this isn't shown. Further, other electrodes could be selected, such as to form a tripole, as explained earlier.

Once stimulation begins (at time = 0), an ECAP will be produced comprising the sum of the action potentials of neural fibers recruited and hence firing in volume 95, and again predominately near cathode electrode E5. As shown in Figure 8A, the ECAP will move through the patient's tissue via neural conduction with speeds of about 5.0 cm/ms, but as noted above this speed can vary depending on the fibers involved in ECAP conduction. Notice that the resulting ECAPs travel in both an orthodromic direction toward the brain (rostrally) and in an antidromic direction toward the bottom of the spinal cord of the patient (caudally).

A single sense electrode (S) has been chosen to sense the ECAP as it moves past, which in this example is electrode E8. Selection of an appropriate sense electrode can be determined by the ECAP algorithm 124 operable in the control circuitry 102 based on a number of factors. For example, it is preferable that a sense electrode S be sensibly chosen with respect to the active electrodes, such that the EM field produced around the active electrodes, i.e., the stimulation artifact 131, will significantly dissipate at the sense electrode by the time the ECAP arrives. It is preferable that the stimulation artifact 131 be as small as possible at the sense electrode when the ECAP is being sensed so that the ECAP can be more easily resolved by the sense amp(s) 110.

The stimulation artifact 131 is shown at sensing electrode E8 in Figure 8B, and as expected is relatively large during the activation of the pulse 133a at the active electrodes. It is assumed in Figure 8B that the stimulation artifact 131 is on the order of milliVolts at E8 during the provision of the pulse 133a. The amplitude of the stimulation artifact 131 depends on its distance from the stimulus, for example, on the distance between cathode electrode E5 and sense electrode E8, with larger distances causing the stimulation artifact 131 amplitude to decline. An example of how maximum stimulation artifact 131 amplitude declines with increasing distance from the stimulus is shown in Figure 9B, which again comprises experimental data taken from porcine tissue.

Even after the active pulse ceases (at t = 0.1 ms), the stimulation artifact 131 does not necessarily drop to zero Volts. This is because there are capacitances inherent in the stimulation of tissue, including in the tissue itself. After the pulse has ended, it can be seen that the amplitude of the stimulation artifact is now on the order of microVolts, and declines over time. Even if sense amplifier 110 strategies (Fig. 7B) are used to eliminate background voltages from the measurement, the stimulation artifact 131 can make sensing of the ECAP difficult at the sensing electrode (E8), particularly if the stimulation artifact 131 is significantly larger than the ECAP at the time that the ECAP is sensed. Simply put, the differential amplifier(s) used in the sense amplifier circuitry 110 may not be able to handle such large difference in voltage while still properly resolving the small-signal neural response.

The ECAP algorithm 124 can enable sensing of the ECAP starting at a particular time to try and mitigate this concern. For example, if it is assumed that the electrodes are spaced at a distance x = 4 mm, and that the ECAP might be expected to have a conduction speed of 5 cm/ms, then the ECAP would reach the sensing electrode E8 (3 * 4 mm = 12 mm away from E5) at 0.24 ms, at which time sensing of the ECAP can be enabled. Such enabling of ECAP sensing can be affected by controlling multiplexer 108 via bus 114 (Fig. 7A) to pass the input from sense electrode E8 to a sense amp 110 at appropriate times. Sensing can be enabled for as long as necessary to detect at least some aspects of the shape and size of the resulting ECAP. For example, sensing can last for a long enough time to allow all polarization and refraction peaks in the ECAP to be detected, which can take as long as up to 3 ms for example. If the total duration of the ECAP is longer than the quiet period between two subsequent pulses, e.g., between pulses 133a and 133b, subsequent pulses 133b may not be enabled until the ECAP measurement has finished.

However, while it is possible to for the ECAP algorithm 124 to enable sensing of ECAPs in a logical manner and at a logical sensing electrode in the IPG's electrode array, it cannot be guaranteed that the stimulation artifact 131 will be sufficiently small at the sensing electrode(s) chosen. To remedy these concerns, the inventors propose new lead designs which are particularly useful to both provide stimulation and to sense neural responses such as Evoked Compound Action Potentials (ECAPs) in a Spinal Cord Stimulation (SCS) system. One or more sensing electrodes on the lead are spaced at significantly larger distances away from the stimulating electrodes, such as at distances of 20 mm to less than 30 mm. Positioning the sensing electrodes at such distances allows for sensing of ECAPs at a sufficient distance away from the stimulating electrodes that ECAP measurements at the sensing electrodes will be less affected by stimulation artifacts that accompany the stimulation. The sensing electrodes may be dedicated to sensing, or may also have the ability to function as stimulating electrodes.

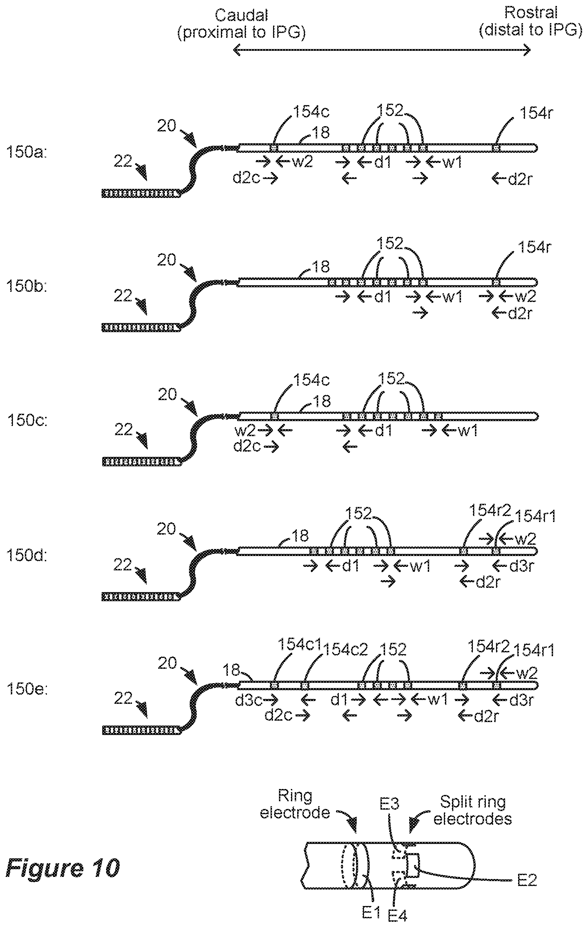

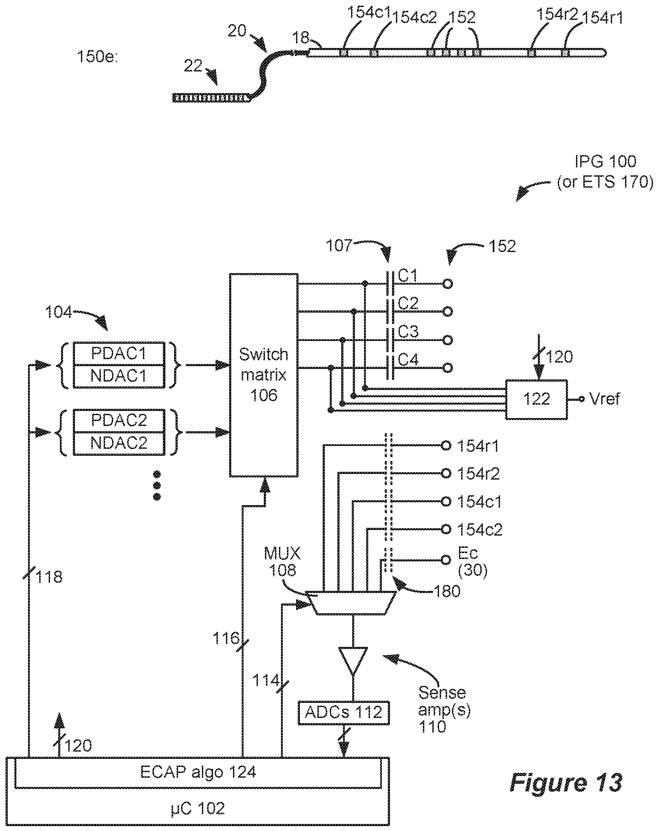

First examples of percutaneous leads 150i particularly useful in sensing ECAPs are shown in Figure 10. Each of the examples in Figure 10 comprise eight-electrode leads, and thus terminate at a set of eight-electrode proximal contacts 22 suitable for insertion into a lead connector 24 having eight header contacts 26 (Figs. 1A and 1B). The eight distal electrodes on the leads 150i comprise stimulating electrodes 152 and sensing electrodes 154. One, more, or all of these electrodes 152 and 154 can in some embodiments both stimulate and sense; in other embodiments electrodes 152 may be dedicated to stimulating while electrodes 154 may be dedicated to sensing, as discussed later with reference to Figure 13. Note that the various examples of the leads subsequently illustrated are not necessarily drawn to scale.

The first example lead 150a comprises six stimulating electrodes 152 and two sensing electrodes 154r and 154c, which in an SCS application would respectively be positioned rostrally (r; most distally from the IPG or ETS) and caudally (c; most proximally) in a patient. In one example, the stimulating electrodes 152 and the sensing electrodes 154 comprise ring electrodes that span 360 degrees around the long axis of the leads 150i. However, split ring electrodes that span less than 360 degrees can also be used for one or more of electrodes 152 and/or 154. Examples of ring electrodes and split ring electrodes as usable on a percutaneous lead are shown at the bottom of Figure 10, and are described in further detail in U.S. Patent Application Publication 2019/0076645. In one example and as relevant to all of the following examples, the stimulating electrodes can comprise split ring electrodes, while the sensing electrodes comprise ring electrodes.

The stimulating electrodes 152 in lead 150a are positioned longitudinally between the sensing electrodes 154r and 154c. The stimulating electrodes 152 are preferably formed and positioned in a manner to span an appreciable longitudinal distance along the lead. This is preferred to provide flexibility in selecting electrodes for stimulation, and to increase the chance that at least some of the stimulating electrodes 152 will be proximate to a neural site of a patient's pain. In one example, the stimulating electrodes 152 can have widths w1 from 1 to 4 mm, and can comprise a spacing d1 between each from 1 to 2.5 mm. Thus, the stimulating electrodes 152 may generally be spaced at their centers from 2 to 6.5 mm. Even though lead 150a is configured for connection to an eight-electrode port, it is not necessary that lead 150a comprise six stimulating electrodes 152. Instead, a fewer number of stimulating electrodes 152 could be used, meaning that some of the proximal contacts 22 will simply be unused.

The sensing electrodes 154r and 154c are preferably spaced at significant distances d2r and d2c from the stimulating electrodes 152. More specifically, sensing electrode 154r is spaced from a closest one of the stimulating electrodes 152 (the right most) by distance d2r, and sensing electrode 154c is spaced from a closest one of the stimulating electrodes 152 (the left most) by distance d2r. This is desired to mitigate the effect that stimulation artifacts might have on ECAPs that are being sensed at the sensing electrodes 154r and 154c. Distances d2r and d2c may be the same, and may depend on the expected speed at which ECAPs formed near the stimulating electrodes 152 will travel on route to the sensing electrodes 154r and 154c. As noted earlier, this ECAP speed depends on many factors, including the types of neural fibers that are recruited by the stimulation. Distances d2r and d2c may also be set with attenuation of the travelling ECAPs in mind, as described earlier with reference to Figure 9A. Distances d2r and d2c may also be determined with the goal of keeping the length of the distal end of lead 150a, i.e., the distance from 154c to 154r, to a manageable distance that is reasonably implantable in a patient's spinal column. Taking all of these factors into consideration, distances d2r and d2c in one example are preferably 20 mm to less than 30 mm. As a comparison of Figures 9A and 9B shows, when the distance between stimulation and sensing is within this range, the ECAP is still relatively sizable (Fig. 9A), while the stimulation artifact 131 is sufficiently small that the ECAP will be resolvable even given the presence of the stimulation artifact. This being said, the distance between stimulation and sensing (d2r or d2c) can be less than 20 mm, or 30 mm or greater, depending on the factors discussed above. For example, d2r and/or d2c can be as large as 100 mm. In another example shown in Figures 9A and 9B, the distance between stimulating and sensing electrodes can be from 15 mm to 40 mm, as this range also shows a reasonable trade off of a relatively large ECAP amplitude and a relatively small stimulation artifact amplitude that the sense simplifier circuitry 110 can resolve. The distance between stimulating and sensing electrodes can also have different ranges, such as from 15 mm to 56 mm, or from 20 mm to less than 56 mm. The sensing electrodes 154 can have widths w2 that are different or similar to the widths w1 of the stimulating electrodes 152.

Because distances d2r and/or d2c can generally range from 20 to 30 mm, while the stimulation electrodes are spaced from each other at a distance d1 of 1 to 2.5 mm, d2r and/or d2c is generally in a range of 8 to 30 times larger than d1.

Leads 150b and 150c in Figure 10 are similar to lead 150a, but have only one sensing electrode 154. Specifically, lead 150b has a single rostrally-positioned sensing electrode 154r and no caudally-positioned sensing electrode, while lead 150c has a single caudally-positioned sensing electrode 154r and no rostrally-positioned sensing electrode. Because only a single sensing electrode 154r or 154c is provided, leads 150b and 150c can support up to seven stimulating electrodes 152 in these eight-electrode lead examples. Providing a sensing electrode at either a rostral position (154r) or a caudal position (154c) can be reasonable, because as noted earlier, an ECAP will travel both rostrally and caudally, and hence only sensing electrode(s) in one of these directions may be required.

Leads 150d and 150e comprise a number of sensing electrodes 154 at either rostral or caudal positions. Lead 150d for example shows use of two rostrally-positioned sensing electrodes 154r1 and 154r2 (and no caudally-positioned sensing electrodes), thus supporting up to six stimulating electrodes 152 in this eight-electrode lead example. Again, the sensing electrodes 154i are spaced from the stimulating electrodes 152 by a distance d2r. More specifically, the closest sensing electrode 154r2 is spaced from a closest one of the stimulating electrodes 152 (the right most) by distance d2r. In lead 150d, the sensing electrodes 154r1 and 154r2 are separated at a distance of d3r, which may range from 1 mm to 30 mm in one example. Providing more than one rostrally- or caudally-positioned sensing electrode 154 can be useful for a number of different reasons. First, it allows for sensing a travelling ECAP at different positions, e.g., at both of 154r1 and 154r2, which can improve the fidelity of the ECAP detected by ECAP algorithm 124. Second, it can allow the ECAP algorithm 124 to detect the speed of travelling ECAP. For example, if the ECAP algorithm 124 knows the distance d3r between the sensing electrodes 154r1 and 154r2 (and possibly also their widths w2), the ECAP algorithm 124 can upon detection of the ECAP at the sensing electrodes determine the speed at which the ECAP is moving. As discussed earlier, knowing the speed at which an ECAP is travelling can be useful to determining the types of fibers that are being recruited by the stimulation, which in turn can be helpful to adjusting the stimulation that the IPG 100 or ETS 170 provides via the ECAP algorithm 124.

Although only two rostrally-positioned sensing electrodes 154r1 and r2 are shown for lead 150d, it should be understood that more than two may be provided (which may further reduce the number of stimulating electrodes 152). Further, although not shown in Figure 10 for simplicity, a lead 150 may be provided that has two or more caudally-positioned sensing electrodes 154 and no rostrally-positioned sensing electrodes.

Lead 150e provides two rostrally-positioned sensing electrodes 154r1 and 154r2 and two caudally-positioned sensing electrodes 154c1 and 154c2, which in this eight-electrode example leaves support for up to four stimulating electrodes 152. However, more than two rostrally-positioned sensing electrodes and/or more than two caudally-positioned sensing electrodes could be used.

The inventors have noticed that the distance between sensing electrodes, e.g., d3r and/or d3c, can be optimized to best sense the neural response such as an ECAP. Experimental data on a different porcine subject is shown in Figure 9C, and shows the ECAP peak-to-peak height that results when the distance between two rostral or caudal sensing electrodes (e.g., 154r1 and 154r2, or 154c1 and 154c2) is varied. As noticed, the signal is maximized when this distance is at about 12 mm. Given this maximum, and what voltages the sense amplifier circuitry 110 can reliably sense, an optimal range of distance between the sensing electrodes can be determined. Assume for example as shown in Figure 9C that a voltage of about 33 microvolts is reliably sensed by the sense amplifier circuitry 110; that is, at this voltage and higher, the sense simplifier circuitry 110 can suitably sense the neural response. This then informs that the sensing electrodes can optimally be spaced at a distance ranging from about 4 mm to 28 mm. In another example shown in Figure 9C, if a voltage of about 48 microvolts is reliably sensed by the sense amplifier circuitry 110, then this informs that the sensing electrodes can optimally be spaced at a distance ranging from about 8mm to 17 mm. Optimal spacing between the sensing electrodes can be defined by different metrics, and can therefore be defined by different range values.

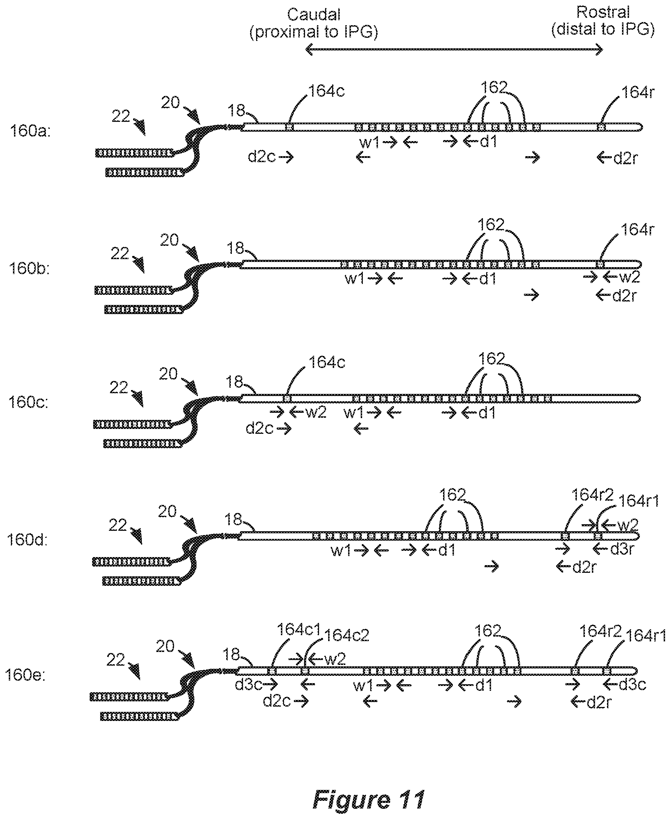

Figure 11 shows examples of percutaneous leads 160i that are generally analogous to leads 150i of Figure 10, which again have one or more sensing electrodes 164 useful to sensing of ECAPs. Each of the examples in Figure 11 comprises sixteen-electrode leads, and thus terminate at sixteen-electrode proximal contacts 22. In the examples as shown, the proximal contacts 22 are divided into two sets of eight-electrode proximal contacts 22, and thus can be connected to two eight-electrode lead connectors 24 of the IPG 100 or ETS 170. However, this is not strictly necessary. Should the IPG 100 or ETS 170 (or their extension cables) comprise sixteen-electrode lead connectors, then the proximal contacts 22 need not be split into two sets as shown, and could instead comprise a single set.

In the examples of Figure 11, each lead comprises sixteen distal electrodes, comprising stimulating electrodes 162 and sensing electrodes 164. One, more, or all of these electrodes 162 and 164 can in some embodiments both stimulate and sense; in other embodiments, electrodes 162 may be dedicated to stimulating while electrodes 164 may be dedicated to sensing.

The first example lead 160a comprises fourteen stimulating electrodes 162 and two sensing electrodes 164r and 164c, which in an SCS application would again respectively be positioned rostrally and caudally in a patient. In one example, the stimulating electrodes 162 and the sensing electrodes 164 comprise ring electrodes that span 360 degrees around the long axis of the leads 160i, although this isn't strictly required.

The stimulating electrodes 162 in lead 160a are positioned longitudinally between the sensing electrodes 164r and 164c. The stimulating electrodes 162 are preferably formed and positioned in a manner to span an appreciable longitudinal distance along the lead, again to provide flexibility in selecting electrodes for stimulation and to increase the chance that at least some of the stimulating electrodes 162 will be proximate to a neural site of a patient's pain. As before, the stimulating electrodes 162 can have widths w1 in one example from 1 to 4 mm, and can comprise a spacing d1 between each from 1 to 2.5 mm. Even though lead 160a is configured for connection to a sixteen-electrode port (or two eight-electrode ports), it is not necessary that lead 160a comprise fourteen stimulating electrodes 162. Instead, a fewer number of stimulating electrodes 162 could be used, meaning that some of the proximal contacts 22 will simply be unused.

The sensing electrodes 164r and 164c are again preferably spaced at significant distances d2r and d2c from the stimulating electrodes 162 to mitigate the effect that stimulation artifacts might have on ECAPs that are being sensed at the sensing electrodes 164r and 164c. Distances d2r and d2c may again be the same, and as before are preferably 20 mm to less than 30 mm.

Leads 160b and 160c in Figure 11 are similar to leads 150b and 150c in that they only one sensing electrode 164. Specifically, lead 160b has a single rostrally-positioned sensing electrode 164r and no caudally-positioned sensing electrode, while lead 160c has a single caudally-positioned sensing electrode 164c and no rostrally-positioned sensing electrode. Because only a single sensing electrode 164r or 164c is provided, leads 160b and 160c can support up to fifteen stimulating electrodes 162 in these sixteen-electrode lead examples.

Leads 160d and 160e comprise a number of sensing electrodes 164 at either rostral or caudal positions. Lead 160d for example shows use of two rostrally-positioned sensing electrodes 164r1 and 164r2 (and no caudally-positioned sensing electrodes), thus supporting up to fourteen stimulating electrodes 162 in this sixteen-electrode lead example. In lead 160d, the sensing electrodes 164r1 and 164r2 are again separated at a distance of d3r, which as described earlier can be useful to sensing an ECAP at multiple locations during its travel as useful to improving fidelity or to determine ECAP speed. Although only two rostrally-positioned sensing electrodes 164r1 and 164r2 are shown in Figure 11, it should be understood that more than two may be provided. Further, although not shown in Figure 11, a lead 160 may be provided that has two or more caudally-positioned sensing electrodes 164 and no rostrally-positioned sensing electrodes.

Lead 160e provides two rostrally-positioned sensing electrodes 164r1 and 164r2 and two caudally-positioned sensing electrodes 164c1 and 164c2, which in this sixteen-electrode example leaves support for up to twelve stimulating electrodes 162. Again, more than two rostrally- or caudally-positioned sensing electrodes 164r and 164c could be used.

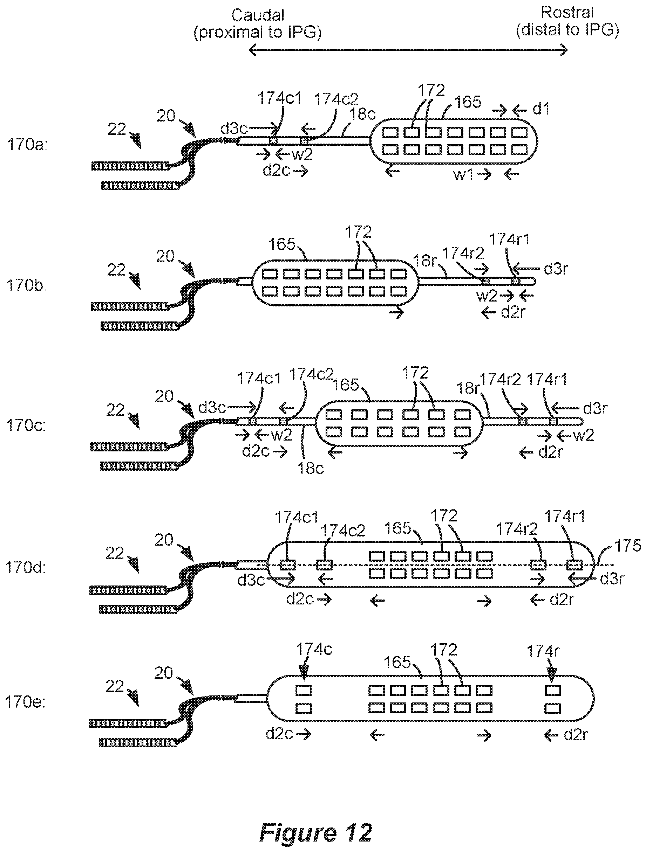

Figure 12 shows example leads 170i involving the use of paddles 165, which like paddle 17 shown earlier (Fig. 1A) comprises a generally flat surface upon which electrodes are positioned. This surface is configured and implanted such that it faces the spinal cord, and generally includes a number of rows of electrodes. Such rows are useful to provide stimulation at different medial-lateral positions along the spinal cord, while the number of electrodes along each row (like the percutaneous leads described earlier) are useful to provide stimulation at different rostral-caudal positions. The illustrated examples show only two rows of electrodes, but one or more than two rows can also be used. The examples shown comprise sixteen-electrode leads, with two sets of eight-electrode proximal contacts 22. However, these leads may support a smaller (e.g., 8) or larger (e.g., 32) number of electrodes.

Example lead 170a includes the paddle 165 at the most distal portion of the lead with respect to the IPG 100, in what would be a rostral position when implanted. Lead 170a also includes a caudal percutaneous lead portion 18c which is more proximal with respect to the IPG 100 than is the paddle 165. In this example, the paddle includes stimulating electrodes 172 (e.g., fourteen of them), while the percutaneous lead portion 18c includes one or more caudally-positioned sensing electrodes 174c1 and 174c2 (e.g., two of them). There are no rostrally positioned sensing electrodes in this example. As with other examples, there could only be one sensing electrode or more than two, but two are shown for simplicity. As before, the stimulating electrodes 172 have a width w1 and are spaced at a distance of d1 with respect to each other in the rostral-caudal direction; these dimensions may be smaller than when percutaneous stimulating electrodes are used to keep the paddle 165 to a manageable size for implantation. The sensing electrode(s) 174 as before can have widths of w2 and be spaced from the stimulating electrodes 172 at a distance d2c, which again is preferably 20 mm to less than 30 mm. If more than one sensing electrode 174 is used, they may be spaced from each other on the percutaneous portion 18c at a distance of d3c as before.

Example lead 170b also includes a paddle 165, but it is not located at the most distal (rostral) portion of the lead. Instead, lead 170a includes a rostral percutaneous lead portion 18r at it most distal position. There are no caudally positioned sensing electrodes in this example. In this example, the paddle includes stimulating electrodes 172 (e.g., fourteen), while the percutaneous lead portion 18r includes one or more rostrally-positioned sensing electrodes 174r1 and 174r2 (e.g., two). The sensing electrode(s) 174 can again be spaced from the stimulating electrodes 172 at a distance d2r, which again is preferably 20 mm to less than 30 mm. If more than one sensing electrode 174 is used, they may be spaced from each other at a distance of d3r as before.

Lead 170c essentially combines the approaches of leads 170a and 170b. It also includes a paddle 165 that is located between both a rostral percutaneous lead portion 18r at it most distal position having one or more sensing electrodes (e.g., 174r1 and 174r2) and a proximal caudal percutaneous lead portion 18c having one or more sensing electrodes (e.g., 174c1 and 174c2). Notice in this sixteen-electrode example that there are twelve stimulating contacts 172 and four sensing contacts 174.

In lead examples 170d and 170e, the sensing electrodes 174 are placed on the paddle 165 itself along with the stimulating electrodes 172. These examples show sensing electrodes 174 being placed in rostral and caudal positions on the paddle. However, although not illustrated for simplicity, the leads 170d and 170e may also contain only rostrally-positioned sensing electrodes 174r or only caudally-positioned sensing electrodes 174c. As before, a distance d2r and/or d2c of 20 mm to less than 30 mm can be used to space the sensing electrodes from the stimulating electrodes, and the sensing electrodes in either the rostral or caudal positions can be spaced from each other at distances of d3r or d3c. The sensing electrodes 174 are placed in different positions in leads 170d and 170e. In lead 170d, the sensing electrodes 174 are centered along a long axis 175 of the lead, which positions may not correspond with the position of any particular row of the stimulating electrodes 172. In lead 170e, the position of the sensing electrodes 174 do correspond to the positions of the rows of stimulating electrodes 172 on the paddle 165, and thus are off center of the long axis 175 just the rows are. Note that while the long axis 175 of any particular lead such as lead 170d is shown is proceeding through the center of the lead, any axis parallel to the center of the lead can comprise a long axis for purposes of this disclosure.

It should be understood that while leads 150i, 160i, and 170i show specific examples each having different aspects, these various aspects can be combined in different fashions, even if all such combinations are not shown.

It has been assumed to this point that any of the various electrodes on leads 150i, 160i, or 170i referred to as stimulating or sensing electrodes can be used to either provide stimulation or to sense neural responses. Such flexible functionality is provided by the IPG 100 or ETS 170 architecture described earlier in Figure 7A, which allows each electrodes to provide stimulation (e.g., if the electrode is selected by the switch matrix 106), or to act as a sensing electrode (e.g., if the electrode is selected by MUX 108). However, the stimulating electrodes (e.g., 152, 162, 172) may be dedicated to only providing stimulation, and the sensing electrodes (e.g., 154i, 164i, and 174i) may be dedicated to only sensing neural responses. This is shown in the IPG or ETS architecture of Figure 13, and using example lead 150e. Any of leads 150i, 160i, and 170i could however have been illustrated, with the architecture adjusted to accommodate the specific number of stimulating and sensing electrodes that each lead has. In this example, stimulating electrodes 152 are connectable to the DACs 104 via the switch matrix 106, and hence can be selected to provide stimulation as described earlier. Stimulating electrodes 152 are not connected to the sense amp(s) 110 used to sense neural responses such as ECAPs. By contrast, sensing electrodes 154i are selectable as sensing electrodes via the sense amp(s) 110, but are not connected to the DACs 104 or the switch matrix 106, and thus cannot provide stimulation. Optionally, the case electrode Ec (30) may also passed to the sense amplifier circuitry, because as noted earlier (Fig. 7B), this voltage can be useful as a reference voltage. Optional capacitors 180 can be provided in the paths from the sensing electrodes 154i to the sense amp(s) 110 to remove DC components of the ECAP signals.

Although particular embodiments have been shown and described, the above discussion should not limit the present invention to these embodiments. Various changes and modifications may be made without departing from the scope of the present invention as defined by the claims.

## Claims

1. A system, comprising:
a lead for a stimulator device, comprising:
a plurality of first electrodes positioned along a long axis of the lead, wherein the first electrodes are spaced from one another by a first distance, wherein the first electrodes are configured to provide stimulation to a patient's tissue to thereby create a neural response;
at least one second electrode positioned distally with respect to the plurality of first electrodes such that one of the at least one second electrode and one of the plurality of first electrodes are closest and spaced from each other by a second distance greater than the first distance, wherein the at least one second electrode is configured to sense the neural response; and
at least one third electrode positioned proximally with respect to the plurality of first electrodes such that one of the at least one third electrode and one of the plurality of first electrodes are closest and spaced from each other by a third distance greater than the first distance, wherein the at least one third electrode is configured to sense the neural response; and
the stimulator device, wherein the lead further comprises at least one set of proximal contacts configured to be received by the stimulator device, wherein the proximal contacts are connected to the plurality of first electrodes, to the at least one second electrode, and to the at least one third electrode by wires,
wherein the stimulator device comprises an architecture, **characterized in that** the architecture is configured to allow the plurality of first electrodes to be selected to only provide stimulation to a patient's tissue, and configured to allow the at least one second electrode and the at least one third electrode to be selected to only sense the neural response.

2. The system of claim 1, wherein the plurality of first electrodes, the at least one second electrode, and the at least one third electrode are located on a percutaneous portion of the lead.

3. The system of claim 2, wherein the at least one second electrode and the at least one third electrode comprise ring electrodes.

4. The system of claim 3, wherein the plurality of first electrodes comprise either ring electrodes or split ring electrodes.

5. The system of any of the preceding claims, wherein the second distance and the third distance are within a range of 15 mm to 100 mm, preferably within a range of 20 mm to less than 30 mm.

6. The system of any of the preceding claims, wherein there is just one second electrode and just one third electrode.

7. The system of any of the preceding claims, wherein there are two or more second electrodes and/or two or more third electrodes.

8. The system of claim 7, wherein a distance between the two or more second electrodes and/or a distance between the two or more third electrodes is 4 to 28 mm.

9. The system of any of the preceding claims, wherein the lead comprises a paddle, wherein the plurality of first electrodes are positioned on the paddle.

10. The system of claim 9, wherein the at least one second electrode is positioned on a percutaneous lead portion positioned distally with respect to the paddle, and wherein the at least one third electrode is positioned on a percutaneous lead portion positioned proximally with respect to the paddle.

11. The system of claim 9, wherein the at least one second electrode and the at least one third electrode are positioned on the paddle.

12. The system of any of the preceding claims, wherein the at least one second electrode and the at least one third electrode are positioned along the long axis.

13. A system, comprising:
a lead for a stimulator device, comprising:
a plurality of first electrodes positioned along a long axis of the lead, wherein the first electrodes are spaced from one another by a first distance, wherein the first electrodes are configured to provide stimulation to a patient's tissue to thereby create a neural response;
at least one second electrode positioned distally with respect to the plurality of first electrodes such that one of the at least one second electrode and one of the plurality of first electrodes are closest and spaced from each other by a second distance greater than the first distance, wherein the at least one second electrode is configured to sense the neural response; and
at least one third electrode positioned proximally with respect to the plurality of first electrodes such that one of the at least one third electrode and one of the plurality of first electrodes are closest and spaced from each other by a third distance greater than the first distance, wherein the at least one third electrode is configured to sense the neural response; and
the stimulator device, wherein the lead further comprises at least one set of proximal contacts configured to be received by the stimulator device, wherein the proximal contacts are connected to the plurality of first electrodes, to the at least one second electrode, and to the at least one third electrode by wires,
wherein the stimulator device comprises an architecture, **characterized in that** the architecture is further configured to allow the plurality of first electrodes to be selected to either provide stimulation to a patient's tissue or to sense the neural response, and configured to allow the at least one second electrode and the least one third electrode to be selected to either provide stimulation to a patient's tissue or to sense the neural response.

## Patentansprüche

1. System, mit:
einer Leitung für eine Stimulationsvorrichtung, die aufweist:
eine Vielzahl von ersten Elektroden, die entlang einer Längsachse der Leitung angeordnet sind, wobei die ersten Elektroden in einem ersten Abstand voneinander beabstandet sind, wobei die ersten Elektroden dafür konfiguriert sind, eine Stimulation für Gewebe eines Patienten bereitzustellen, um eine neurale Reaktion zu erzeugen;
mindestens eine zweite Elektrode, die in Bezug auf die Vielzahl von ersten Elektroden distal angeordnet ist, so dass eine der mindestens einen zweiten Elektrode und eine der Vielzahl von ersten Elektroden am nächsten zueinander und in einem zweiten Abstand voneinander angeordnet sind, der größer ist als der erste Abstand, wobei die mindestens eine zweite Elektrode dafür konfiguriert ist, die neurale Reaktion zu erfassen; und
mindestens eine dritte Elektrode, die in Bezug auf die Vielzahl der ersten Elektroden proximal angeordnet ist, so dass eine der mindestens einen dritten Elektrode und eine der Vielzahl der ersten Elektroden am nächsten zueinander und in einem dritten Abstand voneinander angeordnet sind, der größer ist als der erste Abstand, wobei die mindestens eine dritte Elektrode dafür konfiguriert ist, die neurale Reaktion zu erfassen; und
die Stimulationsvorrichtung, wobei die Leitung ferner mindestens einen Satz von proximalen Kontakten aufweist, die dafür konfiguriert sind, durch die Stimulationsvorrichtung aufgenommen zu werden, wobei die proximalen Kontakte mit der Vielzahl von ersten Elektroden, mit der mindestens einen zweiten Elektrode und mit der mindestens einen dritten Elektrode durch Drähte verbunden sind, wobei die Stimulationsvorrichtung eine Architektur aufweist,
**dadurch gekennzeichnet, dass**
die Architektur dafür konfiguriert ist, zu ermöglichen, dass die Vielzahl der ersten Elektroden derart ausgewählt werden können, dass eine Stimulation nur für das Gewebe eines Patienten bereitgestellt wird, und dafür konfiguriert ist, zu ermöglichen, dass die mindestens eine zweite Elektrode und die mindestens eine dritte Elektrode derart ausgewählt werden können, dass sie nur die neurale Reaktion erfassen.

2. System nach Anspruch 1, wobei die Vielzahl von ersten Elektroden, die mindestens eine zweite Elektrode und die mindestens eine dritte Elektrode an einem perkutanen Abschnitt der Leitung angeordnet sind.

3. System nach Anspruch 2, wobei die mindestens eine zweite Elektrode und die mindestens eine dritte Elektrode Ringelektroden aufweisen.

4. System nach Anspruch 3, wobei die Vielzahl der ersten Elektroden entweder Ringelektroden oder geteilte Ringelektroden aufweisen.

5. System nach einem der vorangehenden Ansprüche, wobei der zweite Abstand und der dritte Abstand in einem Bereich von 15 mm bis 100 mm, vorzugsweise in einem Bereich von 20 mm bis weniger als 30 mm, liegen.

6. System nach einem der vorangehenden Ansprüche, wobei nur eine zweite Elektrode und nur eine dritte Elektrode vorgesehen sind.

7. System nach einem der vorangehenden Ansprüche, wobei zwei oder mehr zweite Elektroden und/oder zwei oder mehr dritte Elektroden vorgesehen sind.

8. System nach Anspruch 7, wobei ein Abstand zwischen den zwei oder mehr zweiten Elektroden und/oder ein Abstand zwischen den zwei oder mehr dritten Elektroden 4 bis 28 mm beträgt.

9. System nach einem der vorangehenden Ansprüche, wobei die Leitung ein Paddel aufweist, wobei die Vielzahl der ersten Elektroden auf dem Paddel angeordnet sind.

10. System nach Anspruch 9, wobei die mindestens eine zweite Elektrode auf einem perkutanen Leitungsabschnitt angeordnet ist, der in Bezug auf das Paddel distal angeordnet ist, und wobei die mindestens eine dritte Elektrode auf einem perkutanen Leitungsabschnitt angeordnet ist, der in Bezug auf das Paddel proximal angeordnet ist.

11. System nach Anspruch 9, wobei die mindestens eine zweite Elektrode und die mindestens eine dritte Elektrode auf dem Paddel angeordnet sind.

12. System nach einem der vorangehenden Ansprüche, wobei die mindestens eine zweite Elektrode und die mindestens eine dritte Elektrode entlang der Längsachse angeordnet sind.

13. System, mit:
einer Leitung für eine Stimulationsvorrichtung, die aufweist:
eine Vielzahl von ersten Elektroden, die entlang einer Längsachse der Leitung angeordnet sind, wobei die ersten Elektroden in einem ersten Abstand voneinander beabstandet sind, wobei die ersten Elektroden dafür konfiguriert sind, eine Stimulation für Gewebe eines Patienten bereitzustellen, um eine neurale Reaktion zu erzeugen;
mindestens eine zweite Elektrode, die in Bezug auf die Vielzahl von ersten Elektroden distal angeordnet ist, so dass eine der mindestens einen zweiten Elektrode und eine der Vielzahl von ersten Elektroden am nächsten zueinander und in einem zweiten Abstand voneinander angeordnet sind, der größer ist als der erste Abstand, wobei die mindestens eine zweite Elektrode dafür konfiguriert ist, die neurale Reaktion zu erfassen; und
mindestens eine dritte Elektrode, die in Bezug auf die Vielzahl der ersten Elektroden proximal angeordnet ist, so dass eine der mindestens einen dritten Elektrode und eine der Vielzahl der ersten Elektroden am nächsten zueinander und in einem dritten Abstand voneinander angeordnet sind, der größer ist als der erste Abstand, wobei die mindestens eine dritte Elektrode dafür konfiguriert ist, die neurale Reaktion zu erfassen; und
die Stimulationsvorrichtung, wobei die Leitung ferner mindestens einen Satz von proximalen Kontakten aufweist, die dafür konfiguriert sind, durch die Stimulationsvorrichtung aufgenommen zu werden, wobei die proximalen Kontakte mit der Vielzahl von ersten Elektroden, mit der mindestens einen zweiten Elektrode und mit der mindestens einen dritten Elektrode durch Drähte verbunden sind, wobei die Stimulationsvorrichtung eine Architektur aufweist,
**dadurch gekennzeichnet, dass**
die Architektur ferner dafür konfiguriert ist, zu ermöglichen, dass die Vielzahl der ersten Elektroden ausgewählt werden können, um entweder eine Stimulation für das Gewebe eines Patienten bereitzustellen oder die neurale Reaktion zu erfassen, und dafür konfiguriert ist, zu ermöglichen, dass die mindestens eine zweite Elektrode und die mindestens eine dritte Elektrode ausgewählt werden können, um entweder eine Stimulation für das Gewebe eines Patienten bereitzustellen oder die neurale Reaktion zu erfassen.

## Revendications

1. Système, comprenant :
un conducteur pour un dispositif stimulateur, comprenant :
une pluralité de premières électrodes positionnées le long d'un axe long du conducteur, dans lequel les premières électrodes sont espacées les unes des autres d'une première distance, dans lequel les premières électrodes sont configurées pour fournir une stimulation au tissu d'un patient pour ainsi créer une réponse neurale ;
au moins une deuxième électrode positionnée de manière distale par rapport à la pluralité de premières électrodes de sorte que l'une parmi l'au moins une deuxième électrode et l'une parmi la pluralité de premières électrodes soient les plus proches et espacées l'une de l'autre d'une deuxième distance supérieure à la première distance, dans lequel l'au moins une deuxième électrode est configurée pour détecter la réponse neurale ; et
au moins une troisième électrode positionnée de manière proximale par rapport à la pluralité de premières électrodes de sorte que l'une parmi l'au moins une troisième électrode et l'une parmi la pluralité de premières électrodes soient les plus proches et espacées l'une de l'autre d'une troisième distance supérieure à la première distance, dans lequel l'au moins une troisième électrode est configurée pour détecter la réponse neurale ; et
le dispositif stimulateur, dans lequel le conducteur comprend en outre au moins un ensemble de contacts proximaux configurés pour être reçus par le dispositif stimulateur, dans lequel les contacts proximaux sont connectés à la pluralité de premières électrodes, à l'au moins une deuxième électrode, et à l'au moins une troisième électrode par des fils, dans lequel le dispositif stimulateur comprend une architecture,
**caractérisé en ce que** l'architecture est configurée pour permettre à la pluralité de premières électrodes d'être sélectionnées pour uniquement fournir une stimulation au tissu d'un patient, et configurée pour permettre à l'au moins une deuxième électrode et à l'au moins une troisième électrode d'être sélectionnées pour uniquement détecter la réponse neurale.

2. Système selon la revendication 1, dans lequel la pluralité de premières électrodes, l'au moins une deuxième électrode, et l'au moins une troisième électrode sont situées sur une partie percutanée du conducteur.

3. Système selon la revendication 2, dans lequel l'au moins une deuxième électrode et l'au moins une troisième électrode comprennent des électrodes annulaires.

4. Système selon la revendication 3, dans lequel la pluralité de premières électrodes comprend soit des électrodes annulaires soit des électrodes annulaires fendues.

5. Système selon l'une quelconque des revendications précédentes, dans lequel la deuxième distance et la troisième distance sont au sein d'une plage de 15 mm à 100 mm, de préférence au sein d'une plage de 20 mm à moins de 30 mm.

6. Système selon l'une quelconque des revendications précédentes, dans lequel il n'y a qu'une seule deuxième électrode et qu'une seule troisième électrode.

7. Système selon l'une quelconque des revendications précédentes, dans lequel il y a deux deuxièmes électrodes ou plus et/ou deux troisièmes électrodes ou plus.

8. Système selon la revendication 7, dans lequel une distance entre les deux deuxièmes électrodes ou plus et/ou une distance entre les deux troisièmes électrodes ou plus est de 4 à 28 mm.

9. Système selon l'une quelconque des revendications précédentes, dans lequel le conducteur comprend une raquette, dans lequel la pluralité de premières électrodes sont positionnées sur la raquette.

10. Système selon la revendication 9, dans lequel l'au moins une deuxième électrode est positionnée sur une partie de conducteur percutanée positionnée de manière distale par rapport à la raquette, et dans lequel l'au moins une troisième électrode est positionnée sur une partie de conducteur percutanée positionnée de manière proximale par rapport à la raquette.

11. Système selon la revendication 9, dans lequel l'au moins une deuxième électrode et l'au moins une troisième électrode sont positionnées sur la raquette.

12. Système selon l'une quelconque des revendications précédentes, dans lequel l'au moins une deuxième électrode et l'au moins une troisième électrode sont positionnées le long de l'axe long.

13. Système, comprenant :
un conducteur pour un dispositif stimulateur, comprenant :
une pluralité de premières électrodes positionnées le long d'un axe long du conducteur, dans lequel les premières électrodes sont espacées les unes des autres d'une première distance, dans lequel les premières électrodes sont configurées pour fournir une stimulation au tissu d'un patient pour ainsi créer une réponse neurale ;
au moins une deuxième électrode positionnée de manière distale par rapport à la pluralité de premières électrodes de sorte que l'une parmi l'au moins une deuxième électrode et l'une parmi la pluralité de premières électrodes soient les plus proches et espacées l'une de l'autre d'une deuxième distance supérieure à la première distance, dans lequel l'au moins une deuxième électrode est configurée pour détecter la réponse neurale ; et
au moins une troisième électrode positionnée de manière proximale par rapport à la pluralité de premières électrodes de sorte que l'une parmi l'au moins une troisième électrode et l'une parmi la pluralité de premières électrodes soient les plus proches et espacées l'une de l'autre d'une troisième distance supérieure à la première distance, dans lequel l'au moins une troisième électrode est configurée pour détecter la réponse neurale ; et
le dispositif stimulateur, dans lequel le conducteur comprend en outre au moins un ensemble de contacts proximaux configurés pour être reçus par le dispositif stimulateur, dans lequel les contacts proximaux sont connectés à la pluralité de premières électrodes, à l'au moins une deuxième électrode, et à l'au moins une troisième électrode par des fils, dans lequel le dispositif stimulateur comprend une architecture,
**caractérisé en ce que** l'architecture est en outre configurée pour permettre à la pluralité de premières électrodes d'être sélectionnées soit pour fournir une stimulation au tissu d'un patient, soit pour détecter la réponse neurale, et configurée pour permettre à l'au moins une deuxième électrode et à l'au moins une troisième électrode d'être sélectionnées soit pour fournir une stimulation au tissu d'un patient, soit pour détecter la réponse neurale.
